# Europäisches Patentamt
## European Patent Office
## Office européen des brevets

(11) Publication number: **0 028 846**
**B1**

(12) **EUROPEAN PATENT SPECIFICATION**

(45) Date of publication of patent specification: **09.12.87**

(51) Int. Cl.⁴: **C 02 F 3/12, C 02 F 3/28, C 12 M 1/16**

(21) Application number: **80200764.1**

(22) Date of filing: **13.08.80**

(54) **Process for preparing biomass attached to a carrier.**

(30) Priority: **07.11.79 NL 7908138**

(43) Date of publication of application:
**20.05.81 Bulletin 81/20**

(45) Publication of the grant of the patent:
**09.12.87 Bulletin 87/50**

(84) Designated Contracting States:
**AT CH DE GB IT LI NL SE**

(56) References cited:
**FR-A-2 391 165**
**GB-A-2 082 164**
**US-A-3 846 289**
**US-A-4 009 098**

**Water Sci. Tech - vol. 15 - 1983 - Pergamon Press Ltd. - John S. Jeris - Industrial waste water treatment using anaerobic fluidized Bed reactors - pages 169-176**

(73) Proprietor: **Gist - Brocades N.V.**
**Wateringseweg 1 P.O. Box 1**
**NL-2600 MA Delft (NL)**

(72) Inventor: **Heijnen, Joseph Johannes**
**Granaathorst 101**
**NL-2592 SR 's-Gravenhage (NL)**

(74) Representative: **Van der Straaten, Jan Anthony et al**
**c/o GIST-BROCADES N.V. Patents and Trademarks Department Martinus Nijhofflaan 2 P.O. Box 1**
**NL-2600 MA Delft (NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The invention relates to a process for preparing biomass attached to a carrier.

In all kinds of biological processes water which contains nutrients is contacted with micro-organisms (so called biomass), the biomass converting the nutrients present in the water. Important processes of this type e.g. are processes for the biological purification of waste water and processes wherein the micro-organisms excrete a desired product as metabolism product.

In the processes applied in practice for the purification of waste water the conversion rate (speed by which the nutrients present in the waste water are converted) calculated per $m^3$ of reactor space, is low on account of the fact that the biomass concentration, which can be obtained in the reactor is low. This is i.a. the case in:

— biological aerobic processes for the COD removal from waste water in a continuous reactor,
— biological aerobic nitrification processes in a continuous reactor,
— biological anaerobic processes for the removal of COD from waste water in a continuous reactor,
— biological anaerobic denitrification processes in a continuous reactor.

Typical biomass concentrations are 1—3 $kg/m^3$ and typical conversion rates are 0.1—3 kg $COD/m^3 \cdot$ day.

The low biomass concentration in those processes is due to the very low settling velocity of micro-organisms, by which from a continuous reactor continuously a discharge of these micro-organisms takes place.

In practice these micro-organisms washed away from the reactor are separated as much as possible by settling, filtration, or centrifugation and subsequently are returned to the reactor. However, these separation technics are expensive (centrifuge) or have their specific restrictions (e.g. floating sludge in the settling tank) and higher biomass concentrations than the mentioned ones are not obtained.

Also in some processes, wherein the micro-organisms form a desired metabolic product which at higher concentration inhibits its own rate of production, the conversion per $m^3$ of reactor volume is low. An example hereof is the production of ethanol from carbohydrate. The inhibition of production leads to low conversion rates per kg of biomass. High conversion rates per $m^3$ of reactor space then only can be obtained by increasing the biomass concentration in the reactor.

From the foregoing it is clear that obtaining a high biomass concentration is necessary in order to obtain high conversion rates in biological processes.

A very promising method for obtaining a high biomass concentration is attaching biomass to a carrier. Such a carrier grown with biomass has a high sedimentation rate if the specific weight of the carrier is sufficiently high. If the settling rate of the carrier grown with biomass is higher than the liquid rate at the reactor outlet, the biomass is not at all washed away.

However, high demands are made upon the adhesion of biomass to such a carrier.

In order to obtain actually a high conversion rate, the other factors which are important for the conversion should not form an impediment.

Thus, in the aerobic purification of waste water at a high biomass concentration $O_2$ should be transferred much quicker; in the anaerobic purification of waste water $CH_4$ and $CO_2$ should be carried off much quicker. The variations in pH, substrate concentrations, temperature or concentration of toxic substances of a waste water, which is introduced into the reactor, should be smoothed out much quicker by adequate mixing of the liquid.

This can only be obtained by dispersing a large amount of mechanical energy in the reactor, which involves high shearing forces in the reactor.

The adhesion of biomass to a carrier now should be such that the biomass still adheres to the carrier in conditions of high shearing forces.

The adhesion of biomass to surfaces is a process which is hardly understood. A survey of the knowledge in this field is to be found in K. C. Marshall "The effect of surfaces on microbial activity", Water Pollution Microbiology 2 (1978) 51. A distinction is made between

(1) Adhesion of biomass to surfaces by weak forces (electrostatic, Van der Waals and hydrophobic inter actions). This adhesion of biomass is so weak, that it is already disturbed by weak shearing forces as e.g. appear in running aerated water. The same forces affect the flocculation of micro-organisms, see C. W. C. Gregor et al "Factors affecting the flocculation of bacteria by chemical additives", Biot. and Bioeng. 11 (1969) and M. W. Tenny et al, "Chemical and autoflocculation of micro-organisms in biological waste water treatment", Biot. and Bioeng. 15 (1973) 1045.

The conclusion is that the adhesion of micro-organisms by these weak forces is lost when high shearing forces play a part. Since, as said before, an economic execution of biological processes with highly increased conversion rates per $m^3$ of reactor volume normally will involve in practice automatically introduction of highly increased shearing forces, the adhesion of biomass through this kind of forces is not interesting.

(2) Adhesion of biomass to surfaces by means of slime films. This adhesion is stronger than the adhesion by the above-mentioned weak forces. In practice this adhesion is well-known and can be seen in the processes like:

(a) Trickle filters, M. F. Kong et al, "Practical design equations for trickling filter process", Biot. and Bioeng. 21 (1979) 417,

(b) Fluidized bed denitrification, J. S. Jeris et al, "high rate biological denitrification using a granular fluidized bed", J. Water Poll. Contr. Fed. 46 (1974), 2118 and Dutch patent application 73 08423.

(c) Fluidized bed BOD-reduction, J. S. Jeris et al, "Biological fluidized bed treatment for BOD and nitrogen removal", J. Water Poll. Contr. Fed. 49 (1977) 816 and Dutch patent application 7401957.

Also when there is question of adhesion by a slime film the biomass comes off from the carrier when strong shearing forces play a part and it is washed away from the reactor. In the fluidized bed processes (b) and (c) use is made of this phenomenon; air is blown in to loose the biofilms in order to prevent blocking of the reactor.

Further, according to the fluidized bed process of US patent No. 4,009,098, for biological aerobic removal of COD, it is as alleged therein possible to achieve interesting rates of conversion by attachment of biomass to carrier while, employing quite high flow rates for the liquid introduced, operating a flexible stirrer mounted in the top of the bed to abrade excessively grown biomass from the carrier surface, and, as preferred, dissolving oxygen in the liquid before it enters the oxidation space.

Also, according to "Suspended-Sludge-on-Solid-Reactor: A New System for the Activated Sludge Process" (by K. Rietema et al in Biotechnology and Bioengineering, Vol. XIII (1971), pages 911—917; published as Communications to the Editor), it was known that it should be advantageous to use sludge in an attached state on suspended carrier, indicating additionally that at least during aerobic COD removal the introduction of air or oxygen in the oxidation space may provide for such a level of stirring motions that it may not be really necessary to employ energy-consuming mechanical stirring additionally. This process however was only applied in excessive sludge producing purification of waste water having a low COD-content in small lab-scale fermentors, while employing a relatively small amount of attached biomass consequent on introducing a relatively very low amount of small-size low-density carrier material (having special surface qualitities) per unit of volume.

However about the conditions which are required for the formation of a biomass attached to a carrier, there is little information.

As a general rule it can be stated that always in a reaction space a granular carrier is contacted with a continuous liquid stream which contains a sufficiently wide flora of micro-organisms and a sufficient amount of nutrients required for the growth and/or preservation of the micro-organisms, until a sufficiently thick layer of micro-organisms is attached to the carrier.

The carrier material is considered as essential by some people (D. W. Levine et al "Optimisation of growth surface parameter in microcarrier culture", Biot. and Bioeng. 21 (1979) 821).

In the above-mentioned known processes wherein use is made of fluidized particles of a carrier with an attached biomass layer, the carrier particles which are seeded with usual bacteries from normal waste water are introduced into a "fluidized bed reactor" and subsequently the carrier is brought in a fluidized condition by an upward stream of waste water wherein before air has been dissolved, whereby a layer of biomass is formed in the carrier particles.

However, as said before, the biomass comes off under conditions of high shearing forces.

In an investigation of the possibility to attach biomass to a carrier such that the biomass does not come off even under conditions of high shearing forces, it appeared that the working conditions are critical.

First a test series was started in order to check whether fluidization per se or the nature of the carrier material was essential for the growth of aerobic active sludge onto a carrier. The tests were carried out in a column reactor with a height of 6 m and a diameter of 25 cm. The temperature was 40°C and the pH-value was adjusted between 6.5 and 8.0. Air was blown in at the bottom of the column through a star-shaped sparger. The air-input was varied between 0.7 and 19.2 Nm³/h. This corresponds with a superficial gas velocity of 0.38—11 cm/s. Under these conditions the power dissipation is 38—1100 W/m³. With amounts of air corresponding to a superficial velocity of 2 cm/s and more, the liquid motion in the column was highly turbulent. Waste water was passed through the bottom of the column and in an amount of 25 l/h. This means a liquid residence time of 11 h in the column.

The COD-concentration of the waste water varied from day to day with as extreme limits 3000 and 12000 mg COD/l. This waste water was freed from all possible suspended organic substance previously.

Subsequently use was made of the following carrier materials:

| | |
|---|---|
| river gravel, grain size | 0.8—1.2 mm |
| silver sand, grain size | 0.1—0.3 mm |
| glass ballotini, grain size | 0.25—0.32 mm |
| glass ballotini, grain size | 0.1—0.12 mm |
| active carbon, grain size | 0.25—1 mm |
| and Eiffel-lava, grain size | <1 mm. |

With each of these carrier materials tests were made using a large range of fluidization patterns varying from no fluidization at all to good fluidization. Each carrier material was tested in the column for 2 or 3 weeks on growth of biomass on the carrier. However, all tests in these series had one disappointing result, that no growth of attached active aerobic sludge took place; herefrom it was concluded, that the nature of the carrier and the test conditions varying from fluidization to no fluidization are no essential factors for adhesion of active aerobic sludge onto a carrier, but that other factors are important.

Further tests learned that for obtaining a biomass film on a carrier, which does not come off at strong shearing forces, it is essential that the carrier is contacted with the liquid while in the liquid 0.1—1.5 kW of mechanical energy per $m^3$ of reactor liquid is dissipated by means of gas bubbling through the liquid and the passage of liquid through the reaction space, and the liquid residence time in the reaction space is kept smaller than the reciprocal maximum growth rate of the micro-organisms.

These requirements might possibly be explained in the following way. The raw waste water, which is passed through the reaction space, contains a great number of various micro-organisms, of which there are some which show adhesive properties to the carrier in the reactor under conditions of high shearing forces, which are present in the column, and which are also capable to decompose the contaminants in the waste water.

By adjusting conditions as to result in a liquid residence time in the reaction space, which is shorter than the reciprocal growth rate of the micro-organisms, while simultaneously dissipating 0.1—1.5 kW mechanical energy per $m^3$ of reactor liquid so that sufficiently high shearing forces are generated in the reactor liquid, one obtains, that all non-adhering micro-organisms are washed away from the reaction space. Then the micro-organisms which can adhere to the carrier, are not overgrown with the non-adhering micro-organisms. They adhere to the carrier, so that they cannot be washed away from the column, and they can consume the substrate, which remains available for the adhering micro-organisms, as the non-adhering micro-organisms do not get the time to grow on the substrate.

The maximum growth rate of the micro-organisms generally depends on the type of micro-organisms, on the type of substrate, on the temperature and on the substrate concentration in the column. Thus the micro-organisms which play a part in the aerobic purification of water and the denitrification micro-organisms grow generally rather quick and the micro-organisms which play a part in the anaerobic water purification and the nitrificating micro-organisms grow much slower. As known, the maximum growth rate of all micro-organisms increases with the temperature according to the Arrhenius-relation.

Now it appeared for the aerobic purification of waste water, that while applying liquids wherein the growth rate of the micro-organisms is not limited by the concentration of nutrients, in practice this growth rate is such, that a liquid residence time in the reactor below 45 minutes leads to a carrier with a good adhering layer of biomass.

If the temperature of the reactor contents is 30—50°C, the residence time of the liquid for forming a layer of aerobic biomass onto the carrier is preferably adjusted at at most 30 minutes. A longer residence time then does not provide a better result, but is less economical.

Similarly, when producing an anaerobic biomass onto a carrier, the residence time is suitably between 1 h and 4 h and if the temperature of the reactor contents is 30—50°C, preferably between 2 h and 3 h.

Adjusting the liquid residence time in the reaction space in principle can be achieved by varying the amount of liquid passed through the reactor.

However, in certain cases practical limits are set hereto.

For instance when producing an aerobic mass onto a carrier not only a relatively short residence time should be adjusted but also sufficient oxygen should be available for the conversion of the COD.

Theoretically per kg of COD about 1.1 kg of oxygen is required and preferably while producing aerobic biomass onto a carrier at least such an amount of air and/or oxygen is introduced, that per kg COD coming into the reaction space, 0.8—1.6 kg is transferred to the reactor liquid.

However, the amount of air and/or oxygen which can be introduced in the reaction space is limited to a maximum.

Therefore, if the available waste water is highly contaminated (has a high COD), as is often the case with industrial waste water, especially from industries where biological materials are treated or biological processes are applied, it is not easy to vary the amount of liquid passed to the reactor, but considering the maximum amount of air and/or oxygen which can be passed through the reactor, the waste water should often be diluted, so that the COD gets a suitable value.

For a practical aerobic reactor, wherein air is introduced and with a liquid residence time in the reactor of 15—30 minutes, e.g. a COD of the waste water between 200 and 500 mg/l is suitable. If the air is replaced by oxygen, then the COD of the waste water can be about 5 times as high, i.e. between 1000 and 2500 mg/l.

In order to obtain a smooth course of the process it is desired that no foaming takes place in the reactor.

Therefore, if necessary, an antifoaming agent can be added. Any known antifoaming agents like silicones, e.g. glycolpolysiloxane, methylphenylpolysiloxane, liquid polydimethylsiloxanes, propylene glycol, triethanolamine, higher alcohols, polyoxyalkylenes are suitable.

The process of the invention can be applied for the production of all kinds of biomass, attached to a carrier, e.g. biomasses as are applied in the biological purification of waste water, aerobic biomass, anaerobic biomass, nitrificating biomass and denitrificating biomass, as well as biomasses which form a desired metabolic product which at higher concentration inhibits its own production rate, like biomass for the production of alcohol.

4

The biomasses on a carrier which are obtained by the process according to the invention allow a considerably higher biomass concentration than is applied in the processes usual until now.

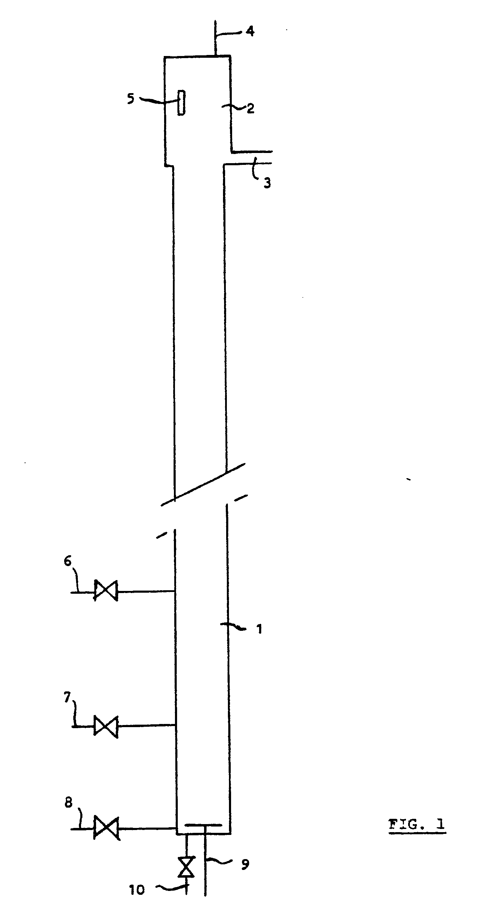

The invention is further elucidated by means of examples wherein use was made of a column as shown in figure 1 of the attached drawings. This figure shows a column 1 which at the top is provided with a settling degassing member 2 which is provided with an overflow 3 and waste gas pipe 4. Also a supply 5 is present by which, if necessary, the anti-foaming agent can be dosed. Waste water is introduced at the bottom of the column at point 6, or at points 6 and 7. Air is blown in through an eight-footed star-shaped sparger 9. Tap water can be introduced in the column via point 8. A sand trap (not shown) is placed behind the column in the outlet for the treated water. The column can be emptied by means of a drain valve 10.

Example I

Use was made of a column according to figure 1 with a height of 6 m and a diameter of 25 cm; the settling/degassing member had a height of 75 cm.

After adding 40 kg of silver sand in the column with a grain size of 0.1—0.3 mm the column was filled with tap water. Subsequently waste water was introduced, in an amount of 25 l/h via point 6. Via point 8 tap water in an amount of 660 l/h was introduced and the pH of the liquid in the column was adjusted to a value between 6.5 and 8.0. The temperature in the column was maintained at 40°C by means of steam injection via point 8. The amount of air which was introduced in a first test was 0.71 $Nm^3$/h (corresponding to 45 $W/m^3$) and in a second test 4.2 $Nm^3$/h (corresponding to 273 $W/m^3$). The total amounts of mechanical energy dissipated in the fluidized bed of these tests can be calculated to 47 $W/m^3$ for the first test and 276 $W/m^3$ for the second test, as the contribution originating from the flow of liquid are approximately 1.6 and 3.3 $W/m^3$ respectively. In the first test the residence time of the liquid was 0.82 hour and in the second test 0.40 hour.

In both tests a considerable growth of biomass on the sand was observable within a few days.

The curves of figure 2 correspond with sample a of the table, and those of figures 3 and 4 with sequentially samples b and c thereof.

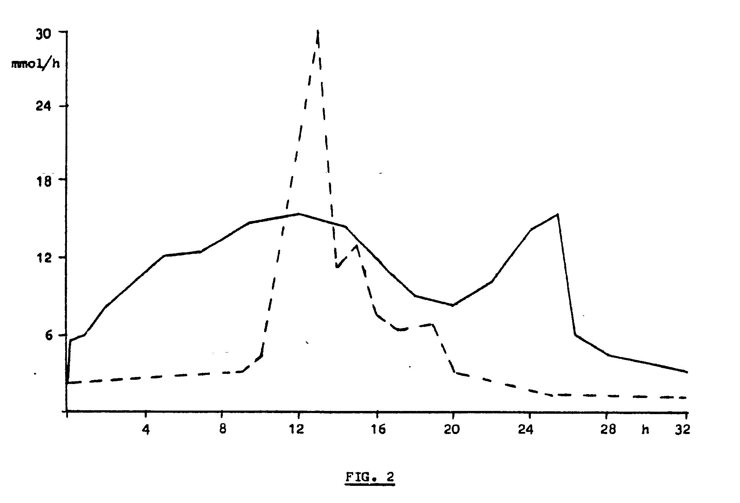

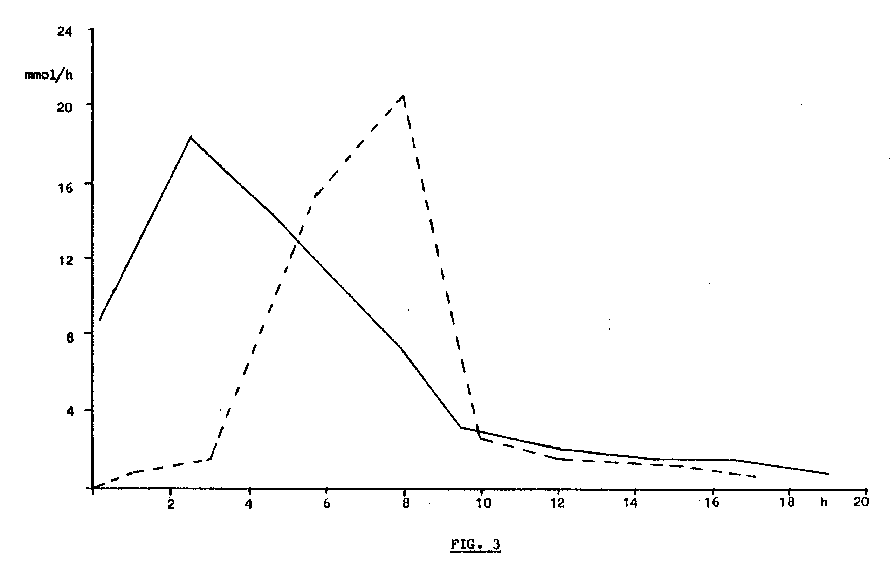

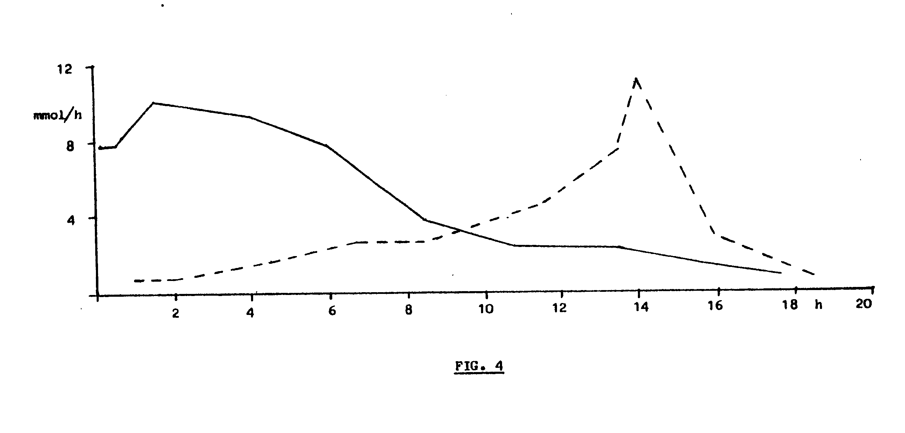

At several times samples of this sand grown with biomass were taken from the column (sample a after the first day of test 1 and samples b and c after the first and second day of test 2) and were thoroughly washed with clean water. Thereafter a test was done with the samples in an Eschweiler-fermentor of 6 l in order to determine the activity of the sand grown with biomass, 25 ml of sample a and 20 ml of sample b or 20 ml of sample c respectively were brought in the fermentor. The Eschweiler fermentor was filled every time with 3 l of waste water and air was passed through in an amount of 200 l/h. The impeller was stirred at a velocity of 200 rpm with sample a, and with a velocity of 300 rpm for samples b and c. The temperature was always 40°C. The results are shown in the graphs of figures 2—4 of the drawing, these graphs show the amount of $O_2$-consumption in mmol/h against the reaction time in h. The drawn curve indicates the $O_2$-consumption as function of time for the fermentor which was seeded with biomass attached to sand.

The dashed curve indicates the $O_2$-consumption for the fermentor which was not seeded with biomass attached to sand (blank). The $O_2$-consumption in the blanks was the result of the growth of bacterias, which are naturally present in the waste water.

It is clear that the tests with a fermentor wherein biomass attached to sand was used show immediately a high $O_2$-consumption with respect to the blank-tests. Also it is remarked that the areas of the curves for the blank tests and for the tests using biomass attached to carrier are roughly equal, or in other words, total $O_2$-consumption is roughly equal. This means that with biomass attached to sand a similar COD-reduction of the waste water is possible as with suspended active sludge.

The samples of biomass attached to sand furthermore were bacteriologically analysed. From these analysis it appeared that large amounts of micro-organisms were present on the sand. From visual observation it appeared that sand grains with a diameter of 200 μm had obtained a diameter of about 300 μm by the attached biomass, in other words, the biofilm was about 50 μm thick. For this film thickness no diffusion limitation is to be expected.

In the following table some analysis results have been collected of the samples of active aerobe sludge on the carrier. All data are based on 1 l of settled sand grains with biofilms in water.

TABLE
Analysis results of biomass attached to sand (silver sand was used with a grain size
of 0.1—0.3 mm, a density of 2.6 g/cm³ and a bulk density of 1.65 g/cm³)

| Sample | Density | g ash/l | g of volatile dry substance per l | N (g/l) | COD (g/l) | P (g/l) | cm³ biofilm per l | Ratio biofilm volume to volatile dry substance cm³/g | Volume ratio of biofilm to sand |
|---|---|---|---|---|---|---|---|---|---|
| a | — | 769 | 95 | 5.00 | — | 2.0 | 364 | 4.0 | 1.23 |
| b | 1.59 | 801 | 61 | 7.16 | 73.3 | 21.8 | 352 | 5.9 | 1.14 |
| c | 1.66 | 610 | 75 | 8.30 | 111.9 | 6.1 | 425 | 5.67 | 1.81 |

From the table it appears that 1 l of sand with biofilm contains about 75 g of biomass dry substance. From the volume ratio of biofilm to sand of 1.2—1.8 it can be calculated that a sand grain with biofilm has 1.3—1.4 times the diameter of the sand grain without a biofilm. At an average grain diameter of 200 μm this means a diameter of 270 μm for the sand grain with a biofilm. This corresponds reasonably with this visual observation.

From the graphs 2—4 it follows that the sand with biofilm had an $O_2$-uptake capacity of about 9 mmol/h for 20 ml of sand with biofilm. For the biomass this means an $O_2$-uptake rate of

$$\frac{9}{0.020 \times 75} = 6 \text{ mmol } O_2/g \cdot h.$$

This is in reasonable conformity with the maximum $O_2$-uptake capacity of suspended sludge of 8 mmol $O_2/g \cdot h.$, found in other tests. This confirms the absence of diffusion-limitation.

Also it follows from the measured N- and COD-concentrations according to the table, that all dry substance consists of organic material. The ratio between biofilm volume and volatile dry substance is also in agreement with this conclusion.

Furthermore it is remarked that sample a was only tested after keeping it for two days at 5°C, so that it seems probable that active sludge attached to a carrier can be preserved for a long time.

From a settling test it appeared furthermore that the settling rate of the sand grains with biofilm was about 50 m/h.

Example II

Use was made of a column according to figure 1 with a height of 6.5 m and a diameter of 25 cm (useful volume 300 l).

In the column 80 kg of silver sand (particle size 0.1—0.3 mm) was introduced.

Via point 6, 40 l/h of waste water with a COD of 6000 mg/l were introduced and via point 8, 760 l/h of tap water were introduced (COD of the mixture 300 mg/l, residence time in the reactor 3/8 h).

Via point 9, 20 Nm³/h of air were introduced (corresponding to an air velocity based on the cross section of the column of 11 cm/s). The total amount of mechanical energy dissipated in the fluidized bed was calculated to 1487 W/m³ as the energies originating from the flow of air and the flow of liquid sequentially were approximately 1480 and 7 W/m³.

After 1 week the sand was overgrown with a layer of biomass and a stationary condition was established.

Analysis of a sample of the sand with biolayer learned that on 1 l of settled sand grains (bulk density 1.65 g/cm³) overgrown with biolayer, 50 g of biomass (calculated as dry substance) were present. The thickness of the biofilm was 40 μm.

The amount of biomass, in the reactor (calculated as dry substance) was 15 g/l.

With this biomass concentration waste water with a COD of 300 mg/l under the above mentioned conditions gave a COD decrease of 70%, at a COD-load of 20 kg/m³ (reactor space) day.

The biomass attached to the carrier remained unaltered; no further growth of biomass took place. Washing away of biomass was not noticed.

Example III

Use was made of a column according to figure 1 with a height of 4 m and a diameter of 7.6 cm (useful volume of 18 l).

In this reactor a test was carried out for the formation of an anaerobic biomass on a carrier.

Herefore 20.6 kg of silver sand (particle size of 0.1—0.3 mm density 2.6 g/cm³, bulk density 1.65 g/cm³) were introduced into the reactor.

Via point 6 waste water with a COD of 6000 mg/l was introduced in an amount of 7.5 l/h (liquid residence time 2.4 h), which corresponds to a COD load of 60 kg COD/m³ · day. These flows of liquid correspond to a dissipation of mechanical energy of approximately 76 W/m³. The pH in the reactor varied between 7—8 and temperature was maintained on 40°C. Gas was developed by anaerobic conversion of COD.

After 6 weeks the sand was overgrown with a layer of biomass and a stationary condition was obtained.

Under the above mentioned conditions, a COD decrease of 60% was obtained after reaching the stationary condition.

Gas development was 15 m³/m³ reactor per day (corresponding to dissipation of mechanical energy of approximately 35 W/m³) and the $CH_4$ content of that gas was 70%. The total amount of mechanical energy dissipated therefore was approximately 111 W/m³.

It appeared that after reaching the stationary condition after 6 weeks sudden changes in the COD of the waste water, changes in the COD-load and a low pH of the waste water had practically no influence on the functioning of the reactor.

7

**0 028 846**

### Claims

1. Process for producing biomass attached to a carrier, wherein in a reaction space a granular carrier is contacted with a continuous stream of liquid which contains a sufficiently wide flora of micro-organisms and sufficient nutrients for the growth and/or preservation of the micro-organisms until a sufficiently thick layer of micro-organisms is attached to the carrier under conditions of exposure to a significant amount of mechanical energy, characterized in that, the carrier is contacted with the liquid stream while in the liquid 0.1—1.5 kW of mechanical energy per $m^3$ of reactor liquid is dissipated by means of gas bubbling through the liquid and the passage of liquid through the reaction space, and the residence time of the liquid in the reaction space is kept lower than the reciprocal maximum growth rate of the micro-organisms.

2. Process according to claim 1, characterized in that, in the production of an aerobic biomass attached to a carrier, while applying liquids wherein the growth rate is not limited by the concentration of nutrients, the residence time of the liquid in the reactor is adjusted below 45 minutes.

3. Process according to claim 2, characterized in that, at a temperature of the reactor contents of 30—50°C, the residence time is at most 30 minutes.

4. Process according to claim 1, characterized in that, in the production of an anaerobic biomass attached to a carrier, while applying liquids wherein the growth rate is not limited by the concentration of nutrients, the residence time of the liquid in the reactor is between 1 h and 4 h.

5. Process according to claim 4, characterized in that, at a temperature of the reactor contents of 30—50°C, the residence time is 2—3 h.

6. Process according to claims 1—3, characterized in that, in the production of an aerobic biomass attached to a carrier, at least such an amount of air and/or oxygen is introduced that per kg COD introduced in the reactor space 0.8—1.6 kg of oxygen is transferred to the reactor liquid.

### Patentansprüche

1. Verfahren zur Herstellung einer auf einen Träger aufgebrachten Biomasse, wobei ein körniger Träger in einem Reaktionsraum mit einem kontinuierlichen Strom von Flüssigkeit, die eine genügend umfangreiche Flora von Mikroorganismen und genügend Nährstoffe für das Wachstum und/oder die Erhaltung der Mikroorganismen enthält, in Berührung gebracht wird, bis unter Bedingungen der Einwirkung einer signifikanten Menge mechanischer Energie eine genügend dicke Schicht von Mikroorganismen auf den Träger aufgebracht ist, dadurch gekennzeichnet, dass der Träger mit dem Flüssigkeitsstrom in Berührung gebracht wird, während 0,1 bis 1,5 kW mechanischer Energie pro $m^3$ Reaktorflüssigkeit mit Hilfe von durch die Flüssigkeit perlendem Gas und dem Durchgang von Flüssigkeit durch den Reaktionsraum in der Flüssigkeit zerstreut wird, und die Verweilzeit der Flüssigkeit in dem Reaktionsraum kürzer als die reziproke maximale Wachstumsgeschwindigkeit der Mikroorganismen gehalten wird.

2. Verfahren nach Anspruch 1, dadurch gekennzeichnet, dass die Verweilzeit der Flüssigkeit in dem Reaktor bei der Herstellung einer auf einen Träger aufgebrachten aeroben Biomasse unter 45 Minuten eingestellt wird, während man Flüssigkeiten anwendet, in denen die Wachstumsgeschwindigkeit nicht durch die Nährstoffkonzentration begrenzt ist.

3. Verfahren nach Anspruch 2, dadurch gekennzeichnet, dass die Verweilzeit bei einer Temperatur des Reaktorinhalts von 30 bis 50°C höchstens 30 Minuten beträgt.

4. Verfahren nach Anspruch 1, dadurch gekennzeichnet, dass die Verweilzeit der Flüssigkeit in dem Reaktor bei der Herstellung einer auf einen Träger aufgebrachten anaeroben Biomasse zwischen 1 Stunde und 4 Stunden liegt, während man Flüssigkeiten anwendet, in denen die Wachstumsgeschwindigkeit nicht durch die Nährstoffkonzentration begrenzt ist.

5. Verfahren nach Anspruch 4, dadurch gekennzeichnet, dass die Verweilzeit bei einer Temperatur des Reaktorinhalts von 30 bis 50°C 2 bis 3 Stunden beträgt.

6. Verfahren nach einem der Ansprüche 1 bis 3, dadurch gekennzeichnet, dass bei der Herstellung einer auf einen Träger aufgebrachten aeroben Biomasse mindestens eine solche Menge Luft und/oder Sauerstoff eingeleitet wird, dass pro kg in den Reaktorraum eingeführtem CSB 0,8 bis 1,6 kg Sauerstoff in die Reaktorflüssigkeit überführt werden.

### Revendications

1. Procédé de préparation d'une biomasse attachée à une matière de support, dans lequel une matière de support granulaire est mise en contact, dans une chambre de réaction, avec un flux continu d'un liquide qui contient une flore de micro-organismes suffisamment large et des agents nutritifs en quantité suffisante pour assurer la croissance et/ou la conservation des micro-organismes jusqu'à ce qu'une couche suffisamment épaisse de micro-organismes soit attachée au support sous l'effet de l'exposition à une quantité suffisante d'énergie mécanique, caractérisé en ce que la matière de support est mise en contact avec le flux de liquide, cependant que de 0,1 à 1,5 kW d'énergie mécanique par $m^3$ de liquide réacteur est dispersée dans le liquide par barbotage de gaz dans le liquide et par passage du liquide à travers la

**0 028 846**

chambre de réaction, et la durée de séjour du liquide dans la chambre de réaction est maintenue inférieure à l'inverse de la vitesse de croissance maximale des micro-organismes.

2. Procédé selon la revendication 1, caractérisé en ce que la durée de séjour du liquide dans le réacteur, dans la préparation d'une biomasse aérobie attachée à une matière de support, est réglée en dessous de 45 minutes, cependant que l'on met en jeu des liquides dans lesquels la vitesse de croissance n'est pas limitée par la concentration des agents nutritifs.

3. Procédé selon la revendication 2, caractérisé en ce que la durée de séjour est de 30 minutes au plus, à une température de 30 à 50°C du contenu du réacteur.

4. Procédé selon la revendication 1, caractérisé en ce que la durée de séjour du liquide dans le réacteur, dans la préparation d'une biomasse anaérobie attachée à une matière de support, est comprise entre 1 heure et 4 heures, cependant que l'on met en jeu des liquides dans lesquels la vitesse de croissance n'est pas limitée par la concentration des agents nutritifs.

5. Procédé selon la revendication 4, caractérisé en ce que la durée de séjour est de 2 à 3 heures, à une température de 30 à 50°C du contenu du réacteur.

6. Procédé selon les revendications 1 à 3, caractérisé en ce que l'on introduit dans la chambre de réaction, dans la préparation d'une biomasse aérobie attachée à une matière de support, au moins une quantité d'air et/ou d'oxygène telle que de 0,8 à 1,6 kg d'oxygène par kg de DCO introduit dans la chambre de réaction soient transférés au liquide réacteur.

9

FIG. 1

FIG. 2

FIG. 3

0 028 846

FIG. 4

0 028 846